# EUROPÄISCHE PATENTSCHRIFT

(11) **EP 1 467 706 B1**
(45) Veröffentlichungstag und Bekanntmachung des Hinweises auf die Patenterteilung: **14.03.2007**
(21) Anmeldenummer: 03704408.8
(22) Anmeldetag: 16.01.2003
(51) Int. Cl.: A61K 9/00

(54) **DERMALES APPLIKATIONSSYSTEM FÜR AMINOLÄVULINSÄURE-DERIVATE**
DERMAL APPLICATION SYSTEM FOR AMINOLEVULINIC ACID-DERIVATIVES
SYSTEME D'ADMINISTRATION DERMIQUE DE DERIVES D'ACIDE AMINOLEVULIQUE

(30) Priorität: 23.01.2002 DE 10202487
(43) Veröffentlichungstag der Anmeldung: 20.10.2004
(73) Patentinhaber: Photonamic GmbH & Co. KG, 20354 Hamburg (DE)
(72) Erfinder: LEE, Geoffrey, 91077 Neunkirchen am Brand (DE); SZEIMIES, Rolf-Markus, 93197 Zeitlarn (DE); KOSCIESSA, Ulrich, 25421 Pinneberg (DE)
(74) Vertreter: UEXKÜLL & STOLBERG
(86) Internationale Anmeldenummer: PCT/EP2003/000406
(87) Internationale Veröffentlichungsnummer: WO 2003/061621

(56) Entgegenhaltungen:
- WO-A-02/05809
- WO-A-95/05813
- WO-A-96/06602

## Beschreibung

Die vorliegende Erfindung betrifft ein dermales Applikationssystem für Aminolävulinsäure-Salze und Aminolävulinsäure-Ester.

Die topische Verwendung von 5-Aminolävulinsäure (δ-ALA; im folgenden als ALA oder 5-ALA bezeichnet) bei der Behandlung oberflächlicher Hauttumoren, insbesondere von Basaliomen, ist erstmals 1990 von Kennedy et al. (J. Photochem. Photobiol. B. 6 (1990) 143-148) beschrieben worden, wobei in erster Linie visuell erkennbare Tumoren lokal mit ALA in Kontakt gebracht werden. Dabei wird ALA selektiv von Tumor-Gewebe aufgenommen und angereichert, wodurch es nur dort zu einer vermehrten Porphyrinbildung und -konzentration führt, während das gesunde Gewebe im wesentlichen unbeeinflußt bleibt. Die Wirkung von ALA beruht auf der Stimulierung der körpereigenen Porphyrinbildung. Da das Porphyrin bei Bestrahlung stark fluoresziert, kann die ALA- bzw. Porphyrin-Anreicherung im Tumorgewebe zur Diagnose präkanzeröser und kanzeröser Läsionen sowie zur photodynamischen Therapie von Tumorerkrankungen ausgenutzt werden.

Der pharmazeutischen Formulierung von Präparaten von ALA und dessen Derivaten sind durch die Instabilität von 5-ALA und ALA-Derivaten in wässriger. Lösung praktische Grenzen gesetzt. So erweist sich ALA bei sehr niedrigem pH-Wert als ausreichend stabil, mit zunehmendem pH-Wert sinkt die Stabilität aber stetig (vergl. Rodriguez et al., S.P.I.E. (Society of Photooptical Instrumentation Engineers) 2371 (1995) 204-209). Eine frische ALA-Lösung weist beispielsweise bei einem etwa physiologischen pH-Wert von 8 nach knapp zwei Wochen nur noch etwa 10% unzersetzten Wirkstoff auf. Aus diesem Grund sind im Handel ALA-Fertigpräparate wie Lösungen und Salben nicht erhältlich, sondern sie müssen, ausgehend von reiner ALA, unmittelbar vor der Anwendung frisch zu bereitet werden und weisen dann eine sehr begrenzte Haltbarkeit auf, die typischerweise weniger als zwei Wochen beträgt.

Eine im Stand der Technik bekannte 5-ALA Methylester-Salbe (Metvix^{®}) ist nach Öffnung ebenfalls nur wenige Tage im Kühlschrank haltbar.

EP 0 704 209 A1 betrifft ALA-enthaltende Zusammensetzungen, insbesondere in Form von Gelen, Emulsionen und dergleichen, mit den oben beschriebenen Nachteilen.

WO95/05813 und WO96/06602 offenbaren Zusammensetzungen zur dermalen Applikation von ALA, die eine vergleichsweise niedrige Freisetzungsgeschwindigkeit für den Wirkstoff aufweisen.

Aufgabe der vorliegenden Erfindung ist es daher, ein Präparat zur Verfügung zu stellen, das ALA-Derivat enthält, in Form einer fertigen Formulierung vorliegt und eine Lagerstabilität bei minimierter Zersetzung des ALA-Derivats aufweist.

Die Aufgabe wird durch das dermale Applikationssystem der vorliegenden Erfindung gelöst. Bei diesem System handelt es sich um ein selbstklebendes Matrixsystem, dessen Polymermatrix kristallines Aminolävulinsäure-Derivat im Teilchengrößenbereich kleiner ca. 200 µm enthält, wobei das Aminolävulinsäure.-Derivat ein Salze oder Ester von ALA ist.

Die genannten Derivate schließen die in WO 96/28412 offenbarten Verbindungen ein, insbesondere Verbindungen der allgemeinen Formel R²₂N-CH₂COCH₂CH₂COOR¹, wobei R¹ einen Alkylrest darstellen kann, der wahlweise durch Hydroxy-, Alkoxy-, Alkyloxy-, Alkoxy-Carbonyloxy-, Amino-, Aryl, Oxo-, oder Fluorgruppe substituiert und wahlweise durch Sauerstoff-, Stickstoff-, Schwefel-, oder Phosphoratome unterbrochen ist, und R² jeweils unabhängig von einander ein Wasserstoffatom oder eine Gruppe wie R¹ darstellt, einschließlich Salze derselben. Gemäß einer bevorzugten Ausführungsform ist die Aryl-Gruppe ein Phenylrest oder ein monogliedriger 5- bis 7-gliedriger heteroaromatischer Rest. R¹ kann eine lineare oder verzweigte, unsubstituierte Alkylgruppe (allgemeine Formel -CₙH₂ₙ₊₁; n ist eine natürliche Zahl von 1 bis 10) sein. Erfindungsgemäß besonders bevorzugt sind 5-Aminolävulinsäuremethylester, 5-Aminolävulinsäureethylester, 5-Aminolävulinsäurepropylester, 5-Aminolävulinsäurebutylester, 5-Aminolävulinsäurepentylester, 5-Aminolävulinsäurehexylester, 5-Aminolävulinsäureheptylester, 5-Aminolävulinsäureoctylester, oder pharmazeutisch akzeptable Salze davon. Die Herstellung dieser Verbindungen ist beispielsweise in WO 96/28412 beschrieben.

Gemäß einer bevorzugten Ausführungsform der Erfindung kann das Applikationssystem ein oder mehrere ALA-Derivate, gegebenenfalls in Kombination mit kristalliner Aminolävulinsäure (ALA), enthalten. Soweit im folgenden von ALA oder ALA-Derivat die Rede ist, können darunter auch die erwähnten Kombinationen verstanden werden.

Im Rahmen der vorliegenden Erfindung wurde überraschenderweise festgestellt, daß eine schnelle Freigabe des ALA-Derivats (ggf. in Kombination mit ALA) durch die Wahl der selbstklebenden polymermatrix nicht nachteilig beeinflußt wird. Aufgrund des ausgewählten Kristallgrößenbereichs wird die Sedimentation der Kristalle des ALA-Derivats (bzw. der in Kombination verwendeten ALA-Kristalle) verhindert, und es herrscht eine homogene Wirkstoff-Verteilung in der Matrix vor.

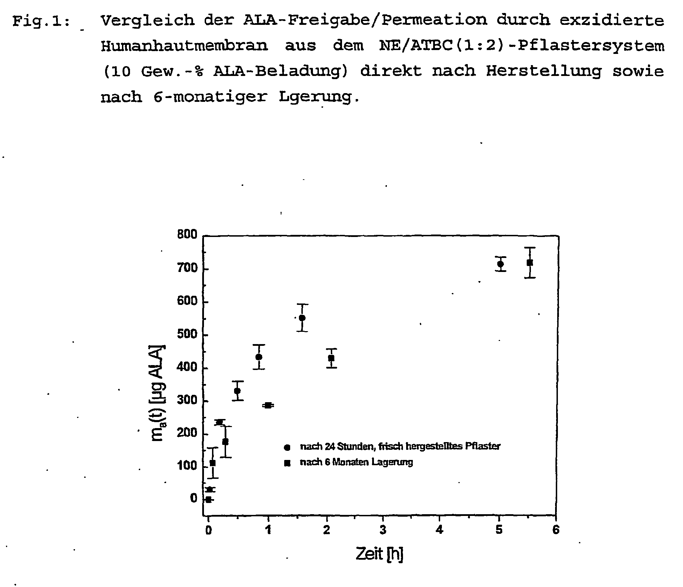

Als dermale Applikationssysteme kommen insbesondere die bislang bekannten Matrixsysteme des PSA-Typs (PSA: Pressure-Sensitive Adhesive) in Betracht, wie sie z.B. in Sugibayashi et al., J. Control. Rel. 29 (1994) 177-185 (vergl. insbesondere Figuren 1a und 1e), oder in der Monographie "Pharmazeutische Technologie: Moderne Arzneiformen" (Kapitel "*Transdermale Therapeutische Systeme*", M. Dittgen; Herausgeber: R. Müller, G. Hildebrand, Wissenschaftliche Verlagsgesellschaft Stuttgart, 1997) beschrieben sind.

Das erfindungsgemäße Applikationssystem enthält vorzugsweise eine wasserdurchlässige Polymermatrix, die besonders bevorzugt nur bedingt wasserdurchlässig ist.

Die selbstklebende Polymermatrix wird bevorzugt aus Polymeren aus der Gruppe bestehend aus
a) Acrylaten,
b) Silikonpolymeren und
c) Polyisobutylen
gebildet, die gegebenenfalls zusätzliche Weichmacher, wie z.B. Citronensäureestern (z.B. Acetyltributylcitrat, ATBC), enthält.

Bei der Wahl der Matrices sind Polymere bervorzugt, die gegenüber ALA nur eine geringes Lösungsvermögen aufweisen, wie z.B. Ethylacrylat-Methylmethacrylat-Copolymerisat (Eudragit NE). Von Vorteil ist ferner eine ausreichende Klebrigkeit, die die Herstellung von selbstklebenden Matrixsystemen ermöglicht, was durch Zusatz von Weichmachern (wie z.B. ATBC) erreicht werden kann.

Als selbstklebende Polymermatrix ist Eudragit NE (NE) mit Acetyltributylcitrat (ATBC) als Weichmacher besonders bevorzugt, insbesondere im Massenverhältnis NE/ATBC von 1:0,5 bis 1:2,5.

Im Rahmen der vorliegenden Erfindung hat sich gezeigt, daß Kristalle des ALA-Derivats (bzw. die in Kombination damit verwendeten ALA-Kristalle) mit einem (mittleren) Durchmesser von weniger als 200 µm, vorzugsweise 20 bis 200 µm, besonders bevorzugt 30 bis 190 µm, von besonderem Vorteil sind. Am meisten sind Kristalle mit einem Durchmesser von 90 bis 160 µm bevorzugt.

Im erfindungsgemäßen dermalen Applikationssystem wird ALA Derivat, ggf. in Kombination mit ALA, vorzugsweise in einer Konzentration von bis zu 50 Gew.-%, insbesondere von mindestens 1 Gew.%, bezogen auf die fertige Polymermatrix eingesetzt. Besonders bevorzugt ist eine ALA- bzw. ALA-Konzentration von etwa 20 Gew.%.

Eine erfindungsgemäß besonders bevorzugte Ausführungsform der Erfindung betrifft ein Applikationssystem, bei dem die Kristalle einen Durchmesser von 90 bis 160 µm besitzen und die Polymermatrix aus Eudragit NE (NE) und Acetyltributylcitrat. (ATBC) im Gewichtsverhältnis NE/ATBC von 1:0,5 bis 1:2,5 besteht, wobei ALA-Derivat bzw. ALA in einer Konzentration von bis zu 50 Gew.-% bezogen auf die fertige Polymermatrix vorliegt.

Die Erfindung betrifft ferner ein Verfahren zur Herstellung dieses Applikationssystems, bei dem man gefriergetrocknetes Eudragit NE (NE) mit Acetyltributylcitrat (ATBC) in Aceton im Massenverhältnis NE/ATBC von 1:0,5 bis 1:2,5 auflöst, man anschließend gemahlenes Aminolävulinsäure-Derivat im Teilchengrößenbereich von 90 bis 160 µm in der Acetonlösung dispergiert und man die so erhaltene Dispersion zu einem dünnen Film auf einen Träger (eine Abdeckfolie) zieht, für 45 Minuten bei 60°C trocknet.

Gemäß einer besonderen Ausführungsform der Erfindung kann man anstelle eines ALA-Derivats auch eine Mischung verschiedener ALA-Derivate oder eine Mischung aus einem oder mehreren ALA-Derivaten und ALA verwenden.

Das hiermit zur Verfügung gestellte Applikationssystem zeichnet sich insbesondere dadurch aus, daß ALA bzw. dessen Derivat im Gegensatz zu Wirkstoffen herkömmlicher Pflastersysteme bzw. transdermaler Applikationssysteme sehr rasch freigesetzt wird und in die Haut eindringen kann. Die hohe Freisetzungsgeschwindigkeit war vor dem Hintergrund der bislang verfügbaren Daten und Kenntnisse auf dem Gebiet der transdermalen therapeutischen Systeme ebensowenig vorhersehbar wie die extrem hohe Lagerstabilität und die dadurch ermöglichte lange Aufbewahrungsdauer (d.h. Lagerfähigkeit bei minimaler Zersetzung von ALA bzw. von dessen Derivat).

Gemäß einer besonderen Ausführungsform der Erfindung werden durch das Applikationssystem innerhalb von 30 Minuten mindestens 30% der in der Polymermatrix dispergierten bzw. suspendierten ALA oder ALA-Derivats freigesetzt. Durch diese schnelle Wirkstoff-Freigabe kann die Einwirkzeit des Applikationssystems gegenüber herkömmlichen Applikationen mittels Salben oder Cremes verkürzt werden, d.h. die Verweilzeit des dermalen Applikationssystems ist deutlich geringer als die Anwendungsdauer bisher verwendeter ALAhaltiger Salben oder Cremes zur Applikation der gleichen Wirkstoffmenge. Gegenüber den erwähnten Salben oder Cremes weist das Applikationssystem ferner den Vorteil auf, daß ALA bzw. ALA-Derivat gezielt in einem scharf begrenzten Hautareal appliziert werden kann, während die bislang im Stand der Technik verwendeten Applikationsformen dies nicht ermöglichen und es daher auch zur Penetration umliegender Hautregionen kommt.

Mit dem dermalen Applikationssystem der vorliegenden Erfindung wird somit erstmals eine stabile, fertige Zubereitung von ALA bzw. eines ALA-Derivats zur Verfügung gestellt, die auch nach Lagerung über einen Zeitraum von einigen Wochen bis mehreren Monaten keine wesentliche Zersetzung von ALA oder des Derivats aufweist. Wie überraschenderweise festgestellt wurde, bestehen bei dem erfindungsgemäßen System unmittelbar nach Herstellung sowie nach sechsmonatiger Lagerung bei 25°C keine wesentlichen unterschiede bezüglich der Wirkstoff-Freigabe und Hautpermeation *in vitro* oder *in vivo.*

Das erfindungsgemäße Applikationssystem ist gegenüber einer Salbe deutlich praktikabler, da es während der Applikationsdauer keine zusätzliche Abdeckung erfordert, um die Kleidung des Patienten zu schützen. Ferner wird keine zusätzliche Lichtschutzschicht benötigt, um den Wirkstoff vor vorzeitiger Bestrahlung zu schützen.

Das genannte Applikationssystem ist zur Verwendung bei der photodynamischen Therapie und/oder Diagnose von präkanzerogenen oder kanzerogenen Läsionen der Haut, insbesondere von Hauttumoren (Basaliomen), besonders geeignet.

Die vorliegende Erfindung wird nachfolgend anhand von Beispielen beschrieben, wobei anstelle von ALA die vorstehend genannten ALA-Derivate, insbesondere 5-Aminolävulinsäuremethylester, 5-Aminolävulinsäureethylester, 5-Aminolävulinsäurepropylester, 5-Aminolävulinsäurebutylester, 5-Aminolävulinsäurepentylester, 5-Aminolävulinsäurehexylester, 5-Aminolävulinsäureheptylester, und 5-Aminolävulinsäureoctylester, verwendet werden.

### BEISPIELE

### Beispiel 1

### Herstellung eines erfindungsgemäßen dermalen Applikationssystems:

Die Pflasterherstellung kann mittels "Solvent Evaporation", "Hot Melt" oder anderer geeigneter Verfahren erfolgen (vgl. z.B. T. Peterson et al., "*Design, Development, Manufacturing and testing of Transdermal Drug Delivery Systems*" in "*Transdermal and Topical Drug Delivery Systems*"; T. Ghosh und W. Pfister, Ed.; Interpharm Press 1997, Buffalo Grove, IL/USA). Dies soll am Beispiel des "Solvent Evaporation"-Verfahrens veranschaulicht werden.

Gemäß einer Ausführungsform der Erfindung wird ALA (Vergleichsbeispiel), ein ALA-Derivat oder eine Mischung derselben, erst gemahlen und klassifiziert, wobei der Teilchengrößenbereich 90 bis 160 µm verwendet wurde. Gefriergetrocknetes Eudragit NE (NE:Trägerpolymer) wird zusammen mit Acetyltributylcitrat (ATBC; Weichmacher) in Aceton im Verhältnis NE/ATBC zwischen 1:0,5 und 1:2,5 aufgelöst. Anschließend erfolgt Zugabe und Dispersion von ALA und/oder ALA-Derivat (s.o.) in Konzentrationen in den fertigen Filmen bis zu 50 Gew.-% (% g/g). Die Zubereitung wird anschließend zum dünnen Film auf eine Abdeckfolie gezogen und 45 Min. bei 60°C getrocknet. Als Abdeckfolie (bzw. Abziehfolie für die Seite des Films, die mit der Haut in Kontakt kommt) eignet sich besonders Melinex 813 bzw. eine silikonisierte Abdeckfolie.

Die Filmklebrigkeit kann durch den Gehalt an ALA und/oder ALA-Derivat (s.o.), durch das verwendete Polymer und den Anteil an Weichmacher (hier ATBC) variiert werden. Die Wirkstoff-Freigabe sowie deren Permeation durch intakte Haut wird durch ATBC beeinflußt (Weichmachereffekt bzw. Permeationsförderungseffekt).

Im Gegensatz zu den herkömmlichen transdermalen therapeutischen systemen (TTS) liegt ALA und/oder ALA-Derivat (s.o.) bei allen Beladungen ≥ ca. 1 Gew.-% (% g/g) größtenteils suspendiert in der lipophilen NE/ATBC-Matrix vor.

### Beispiel 2 - (Vergleichsbeispiel)

Die ALA-Partikel haben eine Größe zwischen ca. 90 und 160 µm und sind im Pflaster homogen verteilt.

Eine homogene Verteilung der ALA-Partikel im fertigen Pflaster erfordert eine Minimalisierung der Sedimentation der Partikel im flüssigen Polymer/Weichmacher/ALA-Zubereitung während der Pflasterherstellung. Dies erfolgt mittels Optimierung der Viskosität der Zubereitung durch Einstellung der Polymerkonzentration. Das Sedimentationsverhalten der ALA-Teilchen wird durch eine Erhöhung der Viskosität reduziert.

| Konzentration von NE[%g/g] in Lösung | Viskosität der Lösung [mPas] | Sinkgeschwindigkeit der ALA-Teilchen* in Lösung [µm/10s] |
|---|---|---|
| 12 | 446 | 396 |
| 18 | 2180 | 119 |
| 24 | 3510 | 3 |

| | | |
|---|---|---|
| * Siebfraktion 60-90 µm | | |

Eine angesetzte NE-Konzentration von ≥25% g/g gewährleistet eine minimale Sedimentationsgeschwindigkeit der ALA-Teilchen.

Für andere Polymermatrices, d.h. andere Polymere, können sich andere ALA-Partikelgrößen als vorteilhaft erweisen, die vom Fachmann gemäß den vorliegenden Informationen und Beispielen auf einfache Weise ermittelt werden können.

### Beispiel 3 - (Vergleichsbeispiel)

ALA im Pflaster ist langzeitstabil.

Die ALA-Freigabe bzw. Hautpermeation aus dem Pflaster direkt nach Herstellung sowie nach 6-monatiger Lagerung bei 25°C zeigt keine wesentlichen Unterschiede (Fig. 1):
Zur Bestimmung des Freigabeprofils wird das Pflaster in eine Diffusionszelle nach Franz (vgl. z.B. K. Tojo, "*Designated Calibration of in vitro Permeation Apparatus*" in "*Transdermal Controlled Systemic Medication*"; Y. Chien, Ed., Marcel Dekker, 1987) bei 33 °C eingespannt. Proben der wässrigen Akzeptorlösung werden nach verschiedenen Zeiten gezogen und deren ALA-Gehalt mittels Fluoreszenz/Derivatisierungs-HPLC-Methode bestimmt.

### Beispiel 4 - (Vergleichsbeispiel)

Durch Anwendung eines Pflasters kann ALA homogen auf Hautläsionen aufgetragen werden.

Die extrem leichte Handhabung der Pflastersysteme stellt gegenüber Salbengrundlagen eine wesentliche Verbesserung für Arzt und Patient dar. Ein entsprechendes ALA-haltiges Pflaster kann auf das zu behandelnde Hautareal recht genau zugeschnitten werden. Dies vermindert die Mitbehandlung der umrandeten Hautfläche, die mit Pflaster nicht beklebt ist.

Durch die Applikation via Pflaster kommt es zu einer scharf begrenzten Applikation, ohne umliegende Hautregionen mit zu penetrieren.

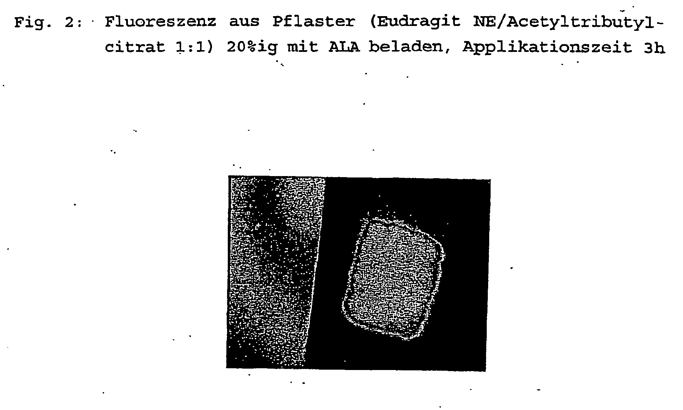

Nach 3h Applikation eines mit 20% ALA-beladenen Pflasters (Eudragit NE/Acetyltributylcitrat 1:1) auf Unterarm wird die Fluoreszenz der Hautareale gemessen. Fig. 2 zeigt, daß die Fluoreszenz scharf auf die Pflastergröße begrenzt ist und ein homogenes Aussehen hat.

### Beispiel 5 - (Vergleichsbeispiel)

Überraschenderweise wird im Gegensatz zu herkömmlichen TTS ein hoher Anteil der Wirkstoffbeladung innerhalb kürzester Zeit freigesetzt.

Konventionelle TTS sind mit einer Vielfalt der eigentlich erforderlichen Dosis des Wirkstoffs beladen (Dittgen, M. "*Transdermale Therapeutische Systeme*" in "*Pharmazeutische Technologie; Moderne Arzneiformen*"; Müller, R. & Hildebrand, G., Ed.; Wissenschaftliche Verlagsgesellschaft Stuttgart, 1997). Diese überhohe Beladung ist notwendig, damit der Wirkstoff über einen Zeitraum von 1-7 Tagen annähernd mit konstanter Geschwindigkeit durch passive Diffusion freigesetzt wird. Während dieser Applikationszeit wird nur ein Anteil <50% der Gesamtwirkstoffbeladung freigesetzt.

**Vergleichsbeispiel 1:** Scopolamin TTS (Ciba) ist ein membrangesteuertes Pflaster und enthält insges. 1,5 mg Scopolamin. Es gibt 170µg Scopolamin/Tag ab und wird 3 Tage getragen. Am Ende des dritten Tages werden daher ca. 30% der Gesamtwirkstoffbeladung freigesetzt.

**Vergleichsbeispiel 2:** Estraderm TTS 25 (Geigy) ist ein klebstoffmembrangesteuertes Pflaster und enthält insges. 2 mg Estradiol. Es setzt 25 µg Estradiol/Tag frei und wird 3-4 Tage getragen. Während dieser Applikationszeit werden daher ca. 5% der Gesamtwirkstoffbeladung freigesetzt.

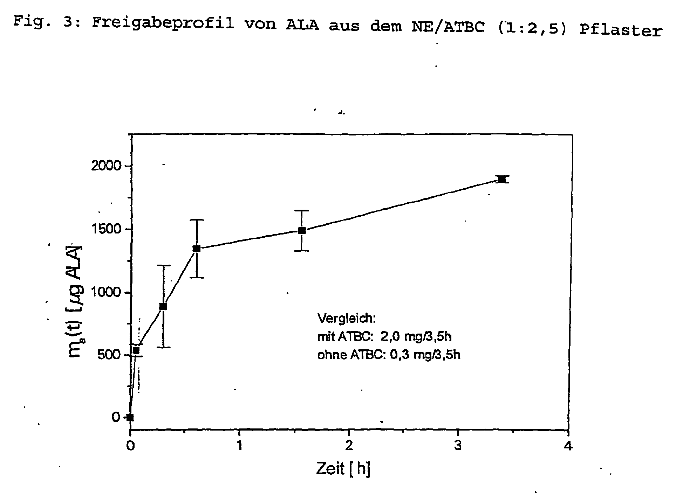

Im Gegensatz zu konventionellen TTS wurde überraschenderweise eine extrem rasche Freigabe der ALA aus dem NE/ATBC-Suspensinspflaster festgestellt. Fig. 3 zeigt das in vitro gemessene Freigabeprofil für ALA aus dem 250 µm-dicken Pflaster aus NE/ATBC (1:2,5) mit 20% g/g ALA-Beladung (Siebfraktion 90-160 µm). Das Pflaster enthielt ins. ca. 4 mg ALA/cm² und hatte bereits nach 1 Minute mehr als 500 µg ALA (entsprechend 12,5% der Gesamtwirkstoffbeladung) freigesetzt. Nach 30 Min. waren mehr als 1,3 mg ALA (entspricht 32 % der Gesamtwirkstoffbeladung) freigesetzt. Die Freigabeprofile wurden wie in Beispiel 3 beschrieben durchgeführt.

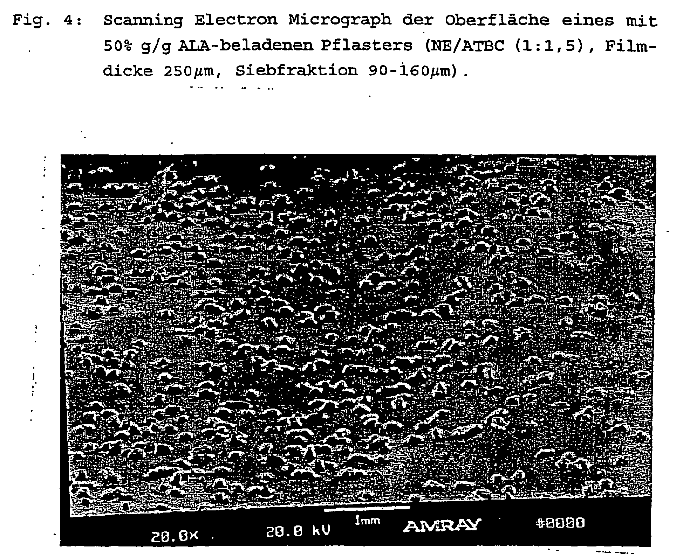

Der Grund für diese extrem rasche Freigabe ist auf den besonderen Aufbau bzw. die Morphologie des dermalen Applikationssystems (Suspensionspflasters) zurückzuführen. Bedingt durch die Anwesenheit suspendierter ALA in der NE/ATBC-Matrix ragen die 90 bis 160 µm großen ALA-Teilchen teilweise durch die Oberfläche der ca. 250 µm dicken Matrix heraus (vgl. Fig. 4).

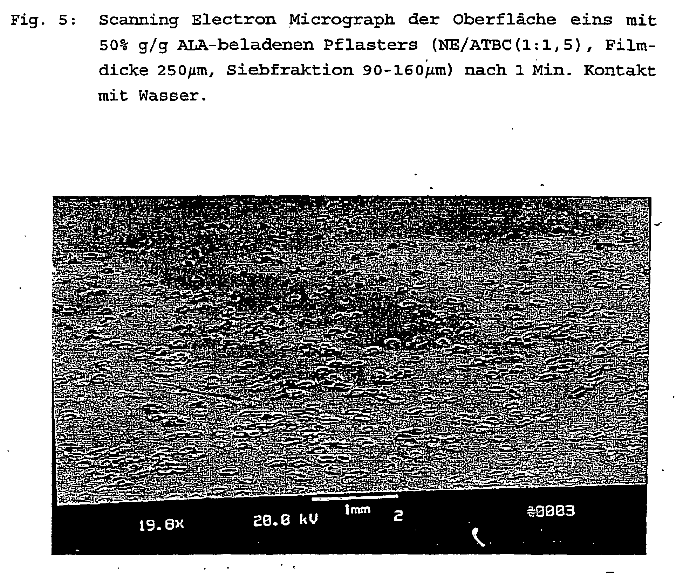

Nach kurzem Kontakt mit dem wässrigen Freigabemedium sind die durch die Oberfläche herausragenden ALA-Teilchen nicht mehr erkennbar (Fig. 5).

Diese unerwartet schnelle "Oberflächenauflösung" der ALA-Teilchen ist eine direkte Konsequenz ihrer hohen Hydrophile und führt zur beobachteten extrem raschen Freigabe der ALA aus dem Pflaster (vergl. Fig. 3).

### Beispiel 6 - (Vergleichsbeispiel)

Die Einwirkzeit des Plastersystems für die Photodynamische Therapie (PDT) ist gegenüber anderen Applikationen mit Salben oder Cremes um ca. 30% verkürzt.

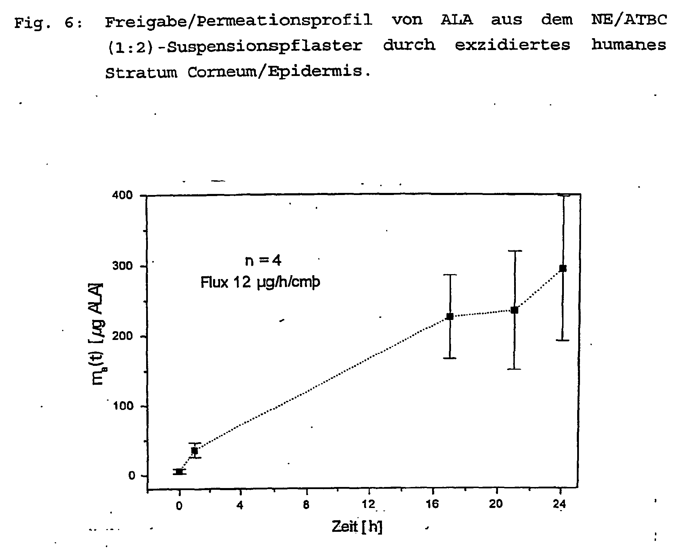

Bestimmung der Permeation von Wirkstoffen durch Membranen aus exzidiertem humanem Stratum Corneum/Epidermis kann mittels Franzzellen durchgeführt werden und stellt ein geeignetes Modell für die in vivo Aufnahme durch menschliche Haut dar. Fig. 6 zeigt das Freigabe/Permeationsprofil für ALA aus dem NE/ATBC (1:2,0)-Pflaster mit Filmdicke 250µm und beladen mit 20% ALA der Siebfraktion 90 bis 160 µm.

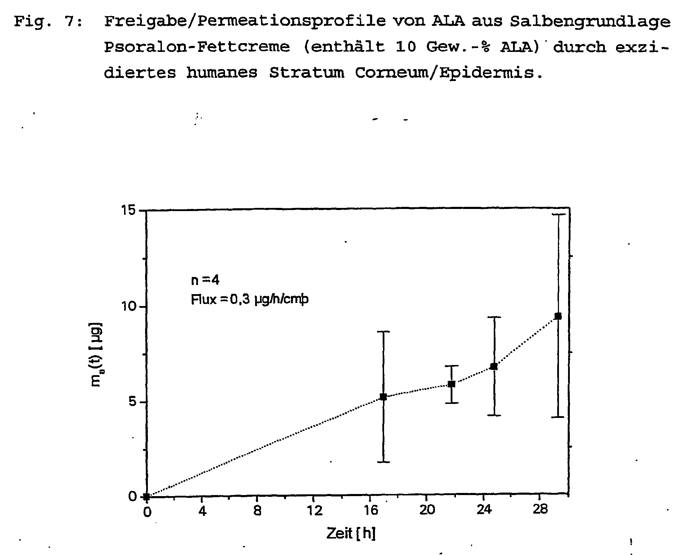

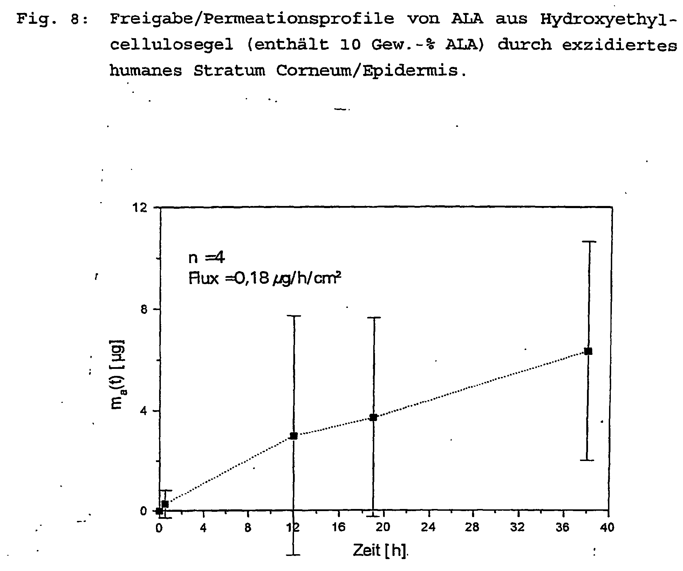

Nach 24 h haben bereits ca. 300 µg ALA die Humanhautmembran passiert. Im Vergleich dazu zeigen Fig. 7 und 8 die deutlich geringere Freigabe/Permeationsprofile der ALA aus den Salbengrundlagen Psoralon-Fettcreme^{®} (enthält 10 Gew.-% ALA) und Hydroxyethylcellulosegel (enthält 10 Gew.-% ALA).

Aus beiden Salbengrundlagen haben nach 24 h weniger als 10 µg ALA die Humanhautmembran passiert. Offensichtlich wird ALA aus dem Pflastersystem rascher und in wesentlich größeren Mengen durch die Humanhautmembran resorbiert. Ein Vergleich der Permeationsraten macht dies quantitativ deutlich:

| System/Grundlage | Permeationsrate [µg/cm²/h] |
|---|---|
| Pflaster NE/ATBC (1:2) | 12 |
| Psoralon-Fettcreme^{®} | 0,3 |
| Hydroxyethylcellulosegel | 0,18 |

Eine raschere bzw. stärkere Wirkung des Pflasters gegenüber der Salbengrundlage während der in vivo PDT ist daher zu erwarten.

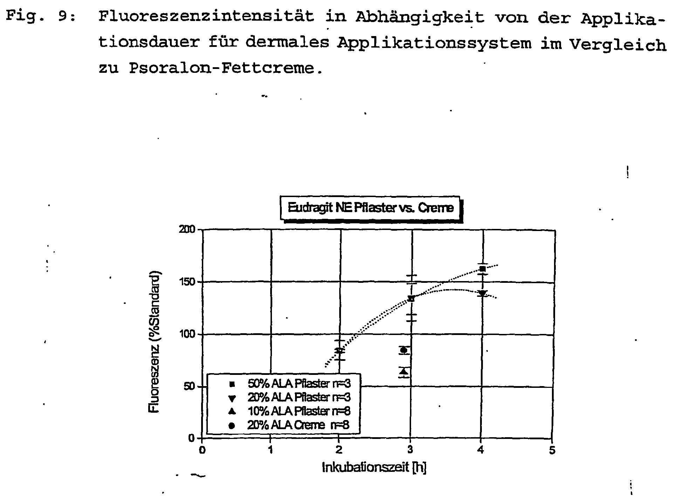

Fig. 9 zeigt die gemessene Fluoreszenzintensität an gesunden Probanden (Unterarm) in Abhängigkeit der Zeit. Die Intensität des mit 20%iger ALA beladenen NE/ATBC (1:2)-Pflasters betrug nach 2 h ca. 80% und nach 3 h ca. 140% einer Standardfluoreszenzzubereitung. Eine 50%ige ALA-Beladung des Pflasters gibt im Vergleich zur 20%igen Beladung der Pflaster keine weitere Steigerung in der Fluoreszenzintensität. Die gemessene Fluoreszenzintensität mit Psoralon-Fettcreme^{®} (20% ALA-Beladung) nach einer Inkubationszeit von 3h ist deutlich niedriger (um ca. 60% als die aus dem NE/ATBC(1:2)-Pflaster (20% ALA-Beladung) bei gleicher Tragezeit (3h). Mit dem erfindungagemäßen Applikationssystem lassen sich somit deutlich kürzere Inkubations-/Applikationszeiten erzielen als mit Applikationsformen wie Salben oder Cremes.

## Patentansprüche

1. Dermales Applikationssystem, das ein selbstklebendes Matrixsystem ist, **dadurch gekennzeichnet, daß** die Polymermatrix ein kristallines Aminolävulinsäure-Salz oder einen kristallinen Aminolävulinsäure-Ester (ALA-Derivat) enthält, wobei die Kristalle des ALA-Derivats eine Größe von weniger als etwa 200 µm aufweisen.

2. Applikationssystem nach Anspruch 1, **dadurch gekennzeichnet, daß** der Aminolävulinsäureester die allgemeinen Formel R²₂N-CH₂COCH₂CH₂COOR¹ aufweist oder ein Salz desselben ist, wobei R¹ ein Alkylrest ist und R² jeweils unabhängig voneinander ein Wasserstoffatom oder eine Gruppe wie R¹ darstellt.

3. Applikationssystem nach Anspruch 2, **dadurch gekennzeichnet, daß** der Alkylrest durch Hydroxy-, Alkoxy-, Alkyloxy-, Alkoxy-Carbonyloxy-, Amino-, Aryl-, Oxo-, oder Fluorgruppen substituiert ist.

4. Applikationssystem nach den Ansprüchen 1 bis 3, **dadurch gekennzeichnet, daß** der Alkylrest durch Sauerstoff-, Stickstoff-, Schwefel-, oder Phosphoratome unterbrochen ist.

5. Applikationssystem nach Anspruch 3, **dadurch gekennzeichnet, daß** die Arylgruppe ein Phenylrest oder ein monocyclischer 5- bis 7-gliedriger heteroaromatischer Rest ist.

6. Applikationssystem nach Anspruch 2, **dadurch gekennzeichnet, daß** R¹ eine unsubstituierte Alkylgruppe ist.

7. Applikationssystem nach den Ansprüchen 1 bis 6, **dadurch gekennzeichnet, daß** die Alkylgruppe 1 bis 10 Kohlenstoffatome aufweist.

8. Applikationssystem nach den Ansprüchen 1 bis 7, **dadurch gekennzeichnet, daß** der Aminoläwlinsäure-Ester 5-Aminolävulinsäuremethylester, 5-Aminolävulinsäureethylester, 5-Aminolävulinsäurepropylester, 5-Aminolävulinsäurebutylester, 5-Aminolävulinsäurepentylester, 5-Aminoläwlinsäurehexylester, 5-Aminolävulinsäureheptylester, 5-Aminolävulinsäureoctylester oder ein Salz desselben ist.

9. Applikationssystem nach den Ansprüchen 1 bis 8, **dadurch gekennzeichnet, daß** das ALA-Derivat eine Mischung verschiedener ALA-Derivate ist.

10. Applikationssystem nach den Ansprüchen 1 bis 9, **dadurch gekennzeichnet, daß** die Polymermatrix wasserdurchlässig ist.

11. Applikationssystem nach den Ansprüchen 1 bis 10, **dadurch gekennzeichnet, daß** die Polymermatrix aus Polymeren aus der Gruppe bestehend aus
a) Acrylaten,
b) Silikonpolymeren und
c) Polyisobutylen
ausgewählt ist.

12. Applikationssystem nach den Ansprüchen 1 bis 11, **dadurch gekennzeichnet, daß** die Kristalle des ALA-Derivats einen mittleren Durchmesser von 30 bis 190 µm haben.

13. Applikationssystem nach Anspruch 12, **dadurch gekennzeichnet, daß** die Kristalle des ALA-Derivats einen mittleren Durchmesser von 90 µm bis 160 µm haben.

14. Applikationssystem nach den Ansprüchen 1 bis 13, **dadurch gekennzeichnet, daß** das Aminolävulinsäure-Derivat in einer Konzentration von 1 bis 50 Gew.-% bezogen auf die fertige Polymermatrix vorliegt.

15. Applikationssystem nach den Ansprüchen 1 bis 14, **dadurch gekennzeichnet, daß** die Kristalle des ALA-Derivats einen Durchmesser von 30 bis 190 µm besitzen und die Polymermatrix aus Eudragit NE (NE) und Acetyltributylcitrat (ATBC) im Gewichtsverhältnis NE/ATBC von 1:0,5 bis 1:2,5 besteht, wobei das ALA-Derivat in einer Konzentration von 1 bis 50 Gew.-% bezogen auf die fertige Polymermatrix vorliegt.

16. Applikationssystem nach Anspruch 15, **dadurch gekennzeichnet, daß** die Kristalle des ALA-Derivats einen Durchmesser von 90 bis 160 µm besitzen.

17. Applikationssystem nach den Ansprüchen 1 bis 16, **dadurch gekennzeichnet, daß** es innerhalb von 30 Minuten mindestens 30 % des ALA-Derivats freisetzt.

18. Applikationssystem nach den Ansprüchen 1 bis 17, **dadurch gekennzeichnet, daß** es ferner kristalline Aminoläwlinsäure (ALA) enthält.

19. Applikationssystem nach Anspruch 18, **dadurch gekennzeichnet, daß** die ALA-Kristalle einen mittleren Durchmesser von 30 bis 190 µm haben.

20. Applikationssystem nach Anspruch 19, **dadurch gekennzeichnet, daß** die ALA-Kristalle einen mittleren Durchmesser von 90 µm bis 160 µm haben.

21. Verfahren zur Herstellung des Applikationssystems nach den Ansprüchen 1 bis 17, **dadurch gekennzeichnet, daß** man gefriergetrocknetes Eudragit NE (NE) mit Acetyltributylcitrat (ATBC) in Aceton im Verhältnis NE/ATBC von 1:0,5 bis 1:2,5 auflöst, man anschließend gemahlenes ALA-Derivat im Teilchengrößenbereich von weniger als etwa 200 µm in der Acetonlösung dispergiert und man die so erhaltene Dispersion zu einem dünnen Film auf eine Abdeckfolie zieht, für 45 Minuten bei 60 °C trocknet.

22. Verfahren nach Anspruch 21, **dadurch gekennzeichnet, daß** man anstelle eines ALA-Derivats eine Mischung verschiedener ALA-Derivate verwendet.

23. Verfahren zur Herstellung des Applikationssystems nach den Ansprüchen 18 bis 20, **dadurch gekennzeichnet, daß** man das Verfahren nach Anspruch 20 durchführt, wobei man anstelle eines ALA-Derivats eine Mischung eines oder mehrerer ALA-Derivate mit ALA verwendet.

24. Applikationssystem nach den Ansprüchen 1 bis 20 zur Anwendung bei der photodynamischen Therapie und/oder Diagnose von präkanzerogenen und kanzerogenen Läsionen der Haut.

25. Applikationssystem nach den Ansprüchen 1 bis 20 zur Anwendung bei der photodynamischen Therapie und/oder Diagnose von Basaliomen.

## Claims

1. Dermal application system, which is a self-adhesive matrix system, **characterized in that** the polymer matrix contains a crystallinic aminolevulinic acid salt or a crystallinic aminolevulinic acid ester (ALA derivative), wherein the crystals of the ALA derivative have a size of less than approximately 200 µm.

2. Application system according to claim 1, **characterized in that** the aminolevulinic acid ester has the general formula R²₂N-CH₂COCH₂COOR¹ or is a salt thereof, wherein R¹ is an alkyl residue and each of R² independently from one another represents a hydrogen atom or a group like R¹.

3. Application system according to claim 2, **characterized in that** the alkyl residue is substituted by hydroxy, alkoxy, alkyloxy, alkoxycarbonyloxy, amino, aryl, oxo, or fluoro groups.

4. Application system according to claims 1 to 3, **characterized in that** the alkyl residue is interrupted by oxygen, nitrogen, sulfur, or phosphorous atoms.

5. Application system according to claim 3, **characterized in that** aryl group is a phenyl residue or a monocyclic 5- to 7-membered heteroaromatic residue.

6. Application system according to claim 2, **characterized in that** R¹ is an unsubstituted alkyl group.

7. Application system according to claims 1 to 6, **characterized in that** the alkyl group has 1 to 10 carbon atoms.

8. Application system according to claims 1 to 7, **characterized in that** the aminolevulinic acid ester is 5-aminolevulinic acid methyl ester, 5-aminolevulinic acid ethyl ester, 5-aminolevulinic acid propyl ester, 5-aminolevulinic acid butyl ester, 5-aminolevulinic acid pentyl ester, 5-aminolevulinic acid hexyl ester, 5-aminolevulinic acid heptyl ester, 5-aminolevulinic acid octyl ester, or a salt thereof.

9. Application system according to claims 1 to 8, **characterized in that** the ALA derivative is a mixture of different ALA derivatives.

10. Application system according to claims 1 to 9, **characterized in that** the polymer system is water-permeable.

11. Application system according to claims 1 to 10, **characterized in that** the polymer matrix is selected from polymers from the group consisting of
a) acrylates,
b) silicon polymers and
c) polyisobutylene.

12. Application system according to claims 1 to 11, **characterized in that** the crystals of the ALA derivative have a mean diameter of 30 to 190 µm.

13. Application system according to claim 12, **characterized in that** the crystals of the ALA derivative have a mean diameter of 90 µm to 160 µm.

14. Application system according to claims 1 to 13, **characterized in that** the aminolevulinic acid derivative is present in a concentration of 1 to 50 wt.% relative to the finished polymer matrix.

15. Application system according to claims 1 to 14, **characterized in that** the crystals of the ALA derivative have a diameter of 30 µm to 190 µm and the polymer matrix consists of Eudragit NE (NE) and acetyl tributyl citrate (ATBC) in the weight ratio NE/ATBC of 1:0.5 to 1:2.5, wherein the aminolevulinic acid derivative is present in a concentration of 1 to 50 wt.% relative to the finished polymer matrix.

16. Application system according to claim 15, **characterized in that** the crystals of the ALA derivative have a diameter of 90 to 160 µm.

17. Application system according to claims 1 to 16, **characterized in that** it releases at least 30% of the ALA derivative within 30 minutes.

18. Application system according to claims 1 to 17, **characterized in that** it further contains crystallinic aminolevulinic acid (ALA).

19. Application system according to claim 18, **characterized in that** the ALA crystals have a mean diameter of 30 to 190 µm.

20. Application system according to claim 19, **characterized in that** the ALA crystals have a mean diameter of 90 µm to 160 µm.

21. Method for the preparation of the application system according to claims 1 to 17, **characterized in that** freeze-dried Eudragit NE (NE) with acetyl tributyl citrate (ATBC) is dissolved in acetone, in the NE/ATBC ratio of 1:0.5 to 1:2.5, after which ground ALA derivative in the particle size range of less than approximately 200 µm is dispersed in the acetone solution, and the dispersion thus obtained is drawn to produce a thin film on a cover foil, and dried for 45 minutes at 60°C.

22. Method according to claim 21, **characterized in that** a mixture of different ALA derivatives is used instead of one ALA derivative.

23. Method for the preparation of the application system according to claims 18 to 20, **characterized in that** the method according to claim 20 is performed, wherein a mixture of one or several ALA derivatives with ALA is used instead of one ALA derivative.

24. Application system according to claims 1 to 20 for use in photodynamic therapy and/or diagnosis of pre-cancerogenic and carcinogenic lesions of the skin.

25. Application system according to claims 1 to 20 for use in photodynamic therapy and/or diagnosis of basaliomas.

## Revendications

1. Système d'administration dermique, qui est un système de matrice autocollant, **caractérisé en ce que** la matrice polymère comprend un sel d'acide aminolévulinique cristallin ou un ester d'acide aminolévulinique cristallin (dérivé ALA), dans lequel les cristaux du dérivé ALA présentent une taille inférieure à environ 200 µm.

2. Système d'administration selon la revendication 1, **caractérisé en ce que** l'ester d'acide aminolévulinique présente la formule générale R²₂N-CH₂COCH₂CH₂COOR¹ ou est un de ses sels, où R¹ est un résidu alkyle et R² représente indépendamment l'un de l'autre un atome d'hydrogène ou un groupe comme défini pour R¹.

3. Système d'administration selon la revendication 2, **caractérisé en ce que** le résidu alkyle est substitué par des groupes hydroxy, alkoxy, alkyloxy, alcoxycarbonyloxy, amino, aryle, oxo ou fluoro.

4. Système d'administration selon la revendication 1 à 3, **caractérisé en ce que** le résidu alkyle est interrompu par un atome d'oxygène, d'azote, de soufre, ou de phosphore.

5. Système d'administration selon la revendication 3, **caractérisé en ce que** le groupe aryle est un résidu phényle ou un résidu hétéroaromatique monocyclique à 5 à 7 chaînons.

6. Système d'administration selon la revendication 2, **caractérisé en ce que** R¹ est un groupe alkyle non substitué.

7. Système d'administration selon les revendications 1 à 6, **caractérisé en ce que** le groupe alkyle comprend de 1 à 10 atomes de carbone.

8. Système d'administration selon les revendications 1 à 7, **caractérisé en ce que** l'ester d'acide aminolévulinique est l'ester méthylique de l'acide 5-aminolévulinique, l'ester éthylique de l'acide 5-aminolévulinique, l'ester propylique de l'acide 5-aminolévulinique, l'ester butylique de l'acide 5-aminolévulinique, l'ester pentylique de l'acide 5-aminolévulinique, l'ester hexylique de l'acide 5-aminolévulinique, l'ester heptylique de l'acide 5-aminolévulinique, l'ester octylique de l'acide 5-aminolévulinique, ou un sel de ces composés.

9. Système d'administration selon les revendications 1 à 8, **caractérisé en ce que** le dérivé ALA est un mélange de différents dérivés ALA.

10. Système d'administration selon les revendications 1 à 9, **caractérisé en ce que**, la matrice polymère est perméable à l'eau.

11. Système d'administration selon les revendications 1 à 10, **caractérisé en ce que**, la matrice polymère est composée de polymères compris dans le groupe des
a) acrylates
b) polymères de silicone, et
c) polyisobutylène.

12. Système d'administration selon les revendications 1 à 11, **caractérisé en ce que** les cristaux du dérivé ALA ont un diamètre moyen de 30 µm à 190 µm.

13. Système d'administration selon la revendication 12, **caractérisé en ce que** les cristaux du dérivé ALA ont un diamètre moyen de 90 µm à 160 µm.

14. Système d'administration selon les revendications 1 à 13, **caractérisé en ce que** le dérivé d'acide aminolévulinique est présent à une concentration de 1 à 50 % en poids par rapport à la matrice préparée.

15. Système d'administration selon les revendications 1 à 14, **caractérisé en ce que** les cristaux du dérivé ALA possède un diamètre de 30 µm à 190 µm et la matrice de polymère consiste en NE Eudragit (NE) et acétyltributylcitrate (ATBC) en rapport en poids NE/ATBC de 1: 0,5 à 1 : 2,5, dans lequel le dérivé ALA est présent en une concentration de 1 à 50 % en poids par rapport à la matrice polymère préparée.

16. Système d'administration selon la revendication 15, **caractérisé en ce que** les cristaux du dérivé ALA possèdent un diamètre de 90 µm à 160 µm.

17. Système d'administration selon les revendications 1 à 16, **caractérisé en ce qu'**au moins 30 % du dérivé ALA sont libérés en l'espace de 30 minutes.

18. Système d'administration selon les revendications 1 à 17, **caractérisé en ce que**, il comprend en outre un acide aminolévulinique cristallin (ALA).

19. Système d'administration selon la revendication 18, **caractérisé en ce que**, les cristaux ALA ont un diamètre moyen de 30 µm à 190 µm.

20. Système d'administration selon la revendication 19, **caractérisé en ce que**, les cristaux ont un diamètre moyen de 90 µm à 160 µm.

21. Procédé de préparation d'un système d'administration selon les revendications 1 à 17, **caractérisé en ce qu'**on dissout du NE Eudragit lyophilisé (NE) avec de l'acétylbutylcitrate (ATBC) dans l'acétone dans un rapport de NE/ATBC de 1 : 0,5 à 1 : 2,5, on disperse ensuite un dérivé ALA en poudre dans le domaine de taille de particules inférieur à environ 200 µm dans la solution d'acétone et on étend la dispersion ainsi obtenue en un film mince sur une feuille de revêtement, on sèche pendant 45 minutes à 60 °C.

22. Procédé selon la revendication 21, **caractérisé en ce qu'**on utilise à la place d'un dérivé ALA un mélange de différents dérivés ALA.

23. Procédé de préparation d'un système d'administration selon les revendications 18 à 20, **caractérisé en ce qu'**on conduit le procédé selon la revendication 20, dans lequel on utilise à la place d'un dérivé ALA un mélange d'un ou de plusieurs dérivés ALA avec ALA.

24. Système d'administration selon les revendications 1 à 20 pour une application lors d'une thérapie photodynamique et/ou d'un diagnostic de lésions précancérogènes ou cancérogènes de la peau.

25. Système d'administration selon les revendications 1 à 20 pour une application lors de la thérapie photodynamique et/ou du diagnostic des épithéliomas basocellulaires.
